# EUROPÄISCHE PATENTSCHRIFT

(11) **EP 1 352 628 B1**
(45) Veröffentlichungstag und Bekanntmachung des Hinweises auf die Patenterteilung: **14.12.2005**
(21) Anmeldenummer: 03011598.4
(22) Anmeldetag: 30.05.1997
(51) Int. Cl.: A61K 7/06

(54) **Wässrige Perlglanzkonzentrate**
Aqueous pearlescent concentrate
Concentré aqueux de lustre nacré

(30) Priorität: 07.06.1996 DE 19622968
(43) Veröffentlichungstag der Anmeldung: 15.10.2003
(62) Teilanmeldung aus: 97925027.1
(73) Patentinhaber: Cognis IP Management GmbH, 40589 Düsseldorf (DE)
(72) Erfinder: Ansmann, Achim, 40699 Erkrath (DE); Kawa, Rolf, 40789 Monheim (DE)

(56) Entgegenhaltungen:
- DE-A- 2 242 541
- DE-A- 2 603 803
- US-A- 3 350 320
- US-A- 5 366 654

## Beschreibung

### Gebiet der Erfindung

Die Erfindung betrifft wäßrige Perlglanzkonzentrate mit einem Gehalt an Fettketonen, Emulgatoren und gegebenenfalls Polyolen, ein Verfahren zu ihrer Herstellung, ein weiteres Verfahren zur Herstellung von perlglänzenden oberflächenaktiven Zubereitungen unter Verwendung der Konzentrate sowie die Verwendung der Fettketone als Perlglanzwachse.

### Stand der Technik

Der weich schimmemde Glanz von Perlen hat auf-den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A. Ansmann und R. Kawa in **Parf.Kosm.** **75****, 578 (1994).**

Der Stand der Technik kennt eine Vielzahl von Formulierungen, die oberflächenaktiven Mitteln den gewünschten Perlglanz verleihen. So sind beispielsweise aus den beiden Deutschen Patentanmeldungen **DE-A1 38 43 572** und **DE-A1 41 03 551** (Henkel) Perlglanzkonzentrate in Form fließfähiger wäßriger Dispersionen bekannt, die 15 bis 40 Gew.-% perlglänzender Komponenten, 5 bis 55 Gew.-% Emulgatoren und 0,1 bis 5 bzw. 15 bis 40 Gew.-% Polyole enthalten. Bei den Perlglanzwachsen handelt es sich um acylierte Polyalkylenglycole, Monoalkanolamide, lineare, gesättigte Fettsäuren oder Ketosulfone. In den beiden Europäischen Patentschriften **EP-B1 0181773 und EP-B1 0 285 389** (Procter & Gamble) werden Shampoozusammensetzungen vorgeschlagen, die Tenside, nicht-flüchtige Silicone und Perlglanzwachse enthalten. Gegenstand der Europäischen Patentanmeldung **EP-A2 0 205 922** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP-B1 0 569 843** (Hoechst) lassen sich nichtionische, fließfähige Perlglanzdispersionen auch erhalten, indem man Mischungen von 5 bis 30 Gew.-% acylierten Polyglycolen und 0,1 bis 20 Gew.-% ausgewählten nichtionischen Tensiden herstellt. Aus der Europäischen Patentanmeldung **EP-A2 0 581 193** (Hoechst) sind ferner fließfähige, konservierungsmittelfreie Perlglanzdispersionen bekannt, die acylierte Polyglycolether, Betaine, Aniontenside und Glycerin enthalten. Schließlich wird in der Europäischen Patentanmeldung **EP-A1 0 684 302** (Th.Goldschmidt) die Verwendung von Polyglycerinestern als Kristallisationshilfsmittel für die Herstellung von Perlglanzkonzentraten vorgeschlagen.

Trotz der Vielzahl von Mitteln besteht im Markt ein ständiges Bedürfnis nach neuen Perlglanzwachsen, die beispielsweise im Gegensatz zu acylierten Polyglycolen keine Ethylenoxideinheiten aufweisen und sich gegenüber den Produkten des Stands der Technik auch bei verminderter Einsatzmenge durch einen brillanten Glanz auszeichnen, die die Mitverwendung kritischer Inhaltsstoffe wie beispielsweise von Siliconen zulassen, ohne daß die Stabilität der Formulierungen beeinträchtigt wird, gleichzeitig über Estergruppen verfügen, damit eine ausreichende biologische Abbaubarkeit gewährleistet ist und die insbesondere in konzentrierter Form noch leicht beweglich und damit handhabbar sind. Die Aufgabe der vorliegenden Erfindung hat somit darin bestanden, neue Perlglanzkonzentrate mit dem geschilderten komplexen Anforderungsprofil zur Verfügung zu stellen.

### Beschreibung der Erfindung

Gegenstand der Erfindung sind wäßrige Perlglanzkonzentrate, enthaltend - bezogen auf den nicht-wäßrigen Anteil -
(a) 1 bis 99,1 Gew.-% Fettketone, mit der Mäßgabe, daß die Stoffe in Summe minde-stens 18, vorzugsweise 24 und insbesondere 32 bis 48 Kohlenstoffatome enthalten,
(b) 0,1 bis 90 Gew.-% anionische, nichtionische, kationische, ampholytische und/oder zwitterionische Emulgatoren sowie
(c) 0 bis 40 Gew.-% Polyole,
mit der Maßgabe, daß sich die Mengenangaben zu 100 Gew.-% ergänzen.

Überraschenderweise wurde gefunden, daß die genannten langkettigen Fettketone ausgezeichnete perlglänzende Eigenschaften besitzen und sich gegenüber den Produkten des Stands der Technik durch eine höhere Brillanz bei geringerer Einsatzmenge, besondere Feinteiligkeit und Lagerstabilität auszeichnen. Die Perlglanzwachse sind leicht biologisch abbaubar, in konzentrierter Form dünnflüssig und erlauben auch die Einarbeitung von problematischen Inhaltsstoffen wie beispielsweise Siliconen in kosmetische Zubereitungen.

### Fettketone

Fettketone, die als Komponente (a) in Betracht kommen, folgen der Formel (II),

**R**^{**2**}**-CO-R**^{**3**} **(II)**

in der R² und R³ unabhängig voneinander für gegebenenfalls hydroxysubstituierte Alkyl- und/oder Alkenylreste mit 1 bis 22 Kohlenstoffatomen stehen, mit der Maßgabe, daß sie in Summe mindestens 18, vorzugsweise 24 und insbesondere 32 bis 48 Kohlenstoffatome aufweisen. Die Ketone können nach Verfahrens des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R² und R³ aber nur um ein Kohlenstoffatom und leiten sich von Fettsäuren mit 16 bis 22 Kohlenstoffatomen ab. Dabei zeichnen sich Caprinon, Lauron und Stearon durch besonders vorteilhafte Perlglanzeigenschaften aus.

Die Einsatzmenge der Fettketone bezogen auf die Konzentrate kann 1 bis 99,9, üblicherweise 5 bis 75, vorzugsweise 10 bis 50 und insbesondere 15 bis 30 Gew.-% betragen.

### Emulgatoren

Die erfindungsgemäßen Perlglanzkonzentrate können als Emulgatoren nichtionogene Tenside aus mindestens einer der folgenden Gruppen enthalten:
(b1) Anlagerungsprodukte von 2 bis 30 Mol Ethylenoxid und/ oder 0 bis 5 Mol Propylenoxid an lineare Fettalkohole mit 8 bis 22 Atomen, an Fettsäuren mit 12 bis 22 C-Atomen, an Alkylphenole mit 8 bis 15 Atomen in der Alkylgruppe und an Triglyceride;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate; (b12) Mischester aus Pentaerythrit, Fettsäuren, Citronensäure und Fettalkohol gemäß **DE-1165574** sowie
(b13) Polyalkylenglycole.

Die Anlagerungsprodukte-von- Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/öder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das KokosacyLaminoethythydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind.

Die erfindungsgemäßen Perlglanzkonzentrate können die Emulgatoren in Mengen von 0,1 bis 90, vorzugsweise 5 bis 50 und insbesondere 10 bis 40 Gew.-% enthalten.

### Polyole

Polyole, die im Sinne der Erfindung als Komponente (c) in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die erfindungsgemäßen Perlglanzkonzentrate können die Polyole, vorzugsweise Glycerin, Propylenglycol, Butylenglycol, Hexylenglycol sowie Polyethylenglycole mit einem durchschnittlichen Molekulargewicht im Bereich von 100 bis 1.0000 in Mengen von 0,1 bis 40, vorzugsweise 0,5 bis 15 und insbesondere 1 bis 5 Gew.-% enthalten.

### Herstellverfahren

In einer bevorzugten Ausführungsform, die ebenfalls Gegenstand der Erfindung ist, erfolgt die Herstellung der Perlglanzkonzentrate, indem man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt. Ferner ist es möglich, eine konzentrierte wäßrige (Anion-)Tensidpaste vorzulegen, das Perlglanzwachs in der Wärme einzurühren und die Mischung anschließend mit weiterem Wasser auf die gewünschte Konzentration zu verdünnen oder das Vermischen in Gegenwart polymerer hydrophiler Verdickungsmittel, wie etwa Hydroxypropylcellulosen, Xanthan Gum oder Polymeren vom Carbomer-Typ durchzuführen.

### Gewerbliche Anwendbarkeit

Die erfindungsgemäßen Perlglanzkonzentrate eignen sich zur Einstellung einer Trübung in oberflächenaktiven Zubereitungen wie beispielsweise Haarshampoos oder manuellen Geschirrspülmitteln. Ein weiterer Gegenstand der Erfindung betrifft daher ein Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C die Perlglanzkonzentrate in einer Menge von 0,5 bis 40, vorzugsweise 1 bis 20 Gew.-% der Zubereitung zusetzt und unter Rühren darin verteilt.

### Tenside

Die oberflächenaktiven Zubereitungen, die in der Regel einen nicht-wäßrigen Anteil im Bereich von 1 bis 50 und vorzugsweise 5 bis 35 Gew.-% aufweisen, können nichtionische, anionische, kationische und/oder amphotere bzw. amphotere Tenside enthalten, deren Anteil an den Mitteln üblicherweise bei etwa 50 bis 99 und vorzugsweise 70 bis 90 Gew.-% beträgt. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid-(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Die gleichen Tenside können auch unmittelbar zur Herstellung der Perlglanzkonzentrate eingesetzt werden, die anionischen Tenside eignen sich auch als Emulgatoren. In diesem Zusammenhang ist der Einsatz von Alkylethersulfaten als anionische Emulgatoren bevorzugt.

### Hilfs- und Zusatzstoffe

Die oberflächenaktiven Zubereitungen, denen die erfindungsgemäßen Perlglanzkonzentrate zugesetzt werden, können weitere Hilfs- und Zusatzstoffe enthalten, wie beispielsweise Ölkörper, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und-Ammoniumchlorid.

Geeignete **kationische Polymere** sind beispielsweise kationische Cellulosederivate, kationische Stärke, Copolymere von Diallylammoniumsalzen und Acrylamiden, quatemierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quatemierte Weizenpolypeptide, Polyethylenimin, kationische Siliconpolymere wie z.B. Amidomethicone oder Dow Coming, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quatemierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Im Sinne der Erfindung können neben den Fettstoffen auch weitere bekannte **Perlglanzwachse** wie insbesondere Mono- und Difettsäureester von Polyalkylenglycolen, Partial- und Triglyceride sowie Ester von Fettalkoholen mit mehrwertigen Carbonsäuren bzw. Hydroxycarbonsäuren eingesetzt werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel"** der **Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Ein letzter Gegenstand der Erfindung betrifft schließlich die Verwendung der genannten Fettstoffe als Perlglanzwachse zur Herstellung von oberflächenaktiven Zubereitungen.

### Beispiele

Das erfindungsgemäße Perlglanzkonzentrat R1 sowie die Vergleichsmischung R2 wurden 14 Tage bei 40°C gelagert und die Viskosität nach der Brookfield-Methode in einem RVT-Viskosimeter (23°C, 10 Upm, Spindel 5) bestimmt. Anschließend wurde eine wäßrige Haarshampooformulierung durch Vermischen der Einsatzstoffe bei 20°C zubereitet, das 2 g des Perlglanzkonzentrates R1, 15 g Kokosfettalkohol+2EO-sulfat-Natriumsalz, 3 g Dimethylpolysiloxan, 5 g Kokosalkylglucosid und 1,5 g eines Esterquats (Wasser ad 100 Gew.-%) enthielt. Die Feinteiligkeit der Perlglanzkristalle im Haarshampoo wurde unter dem Mikroskop visuell auf einer Skala von 1 = sehr feine Kristalle bis 5 = grobe Kristalle beurteilt. Die Beurteilung des Perlglanzes erfolgte ebenfalls auf einer Skala von 1 = brillant bis 5 = stumpf; die Trübung wurde visuell bestimmt und mit (+) = trüb oder (-) = trübungsfrei beurteilt. Die Zusammensetzungen und Ergebnisse sind in Tabelle 1 zusammengefaßt; alle Mengenangaben verstehen sich als Gew.-%

**Tabelle 1**

| **Zusammensetzung und Performance von Perlglanzkonzentraten** | | |
|---|---|---|
| **Zusammensetzung** | **R1** | **R2** |
| C_{32/48}-Fettalkohol | - | - |
| Stearon | 25 | - |
| Distearylether | - | - |
| Mono/Distearylcarbonat | - | - |
| Ethylenglycoldistearat | - | 25 |
| Kokosalkohol+4EO | 5 | 5 |
| Kokosalkylglucosid | 9 | 9 |
| Kokosfettsäurebetain | 5 | 5 |
| Kokosalkohol+2EO-sulfat-Na-Salz | - | - |
| Glycerin | 5 | 5 |
| Propylenglycol/Butylenglycol (1:5) | - | - |
| Wasser | ad 100 | |

| ***Viskosität der Konzentrate [mPas]*** | | |
|---|---|---|
| - nach 1 d, 40°C | 8.200 | 9.500 |
| - nach 14 d, 40°C | 7.800 | 7.200 |

| ***Perlglanz in der Formulierung*** | | |
|---|---|---|
| - Brillanz | 1,5 | 2,0 |
| - Feinteiligkeit | 1,5 | 3,0 |
| - Trübung | - | + |

## Patentansprüche

1. Wäßrige Perlglanzkonzentrate, enthaltend - bezogen auf den nicht-wäßrigen Anteil -
(a) 1 bis 99,9 Gew.-% Fettketone, mit der Maßgabe, daß sie in Summe mindestens 18 Kohlenstoffatome enthalten,
(b) 0,1 bis 90 Gew.-% anionische, nichtionische, kationische, ampholytische und/oder zwitterionische Emulgatoren sowie
(c) 0 bis 40 Gew.-% Polyole,
mit der Maßgabe, daß sich die Mengenangaben zu 100 Gew.-% ergänzen.

2. Pedglanzkonzentrate nach Anspruch 1, **dadurch gekennzeichnet, daß** sie als Komponente (a) Fettketone der Formel (II) enthalten,
**R**^{**2**}**-CO-R3 (II)**
in der R² und R³ unabhängig voneinander für gegebenenfalls hydroxysubstituierte Alkyl- und/oder Alkenylreste mit 1 bis 22 Kohlenstoffatomen stehen, mit der Maßgabe, daß sie in Summe mindestens 18 Kohlenstoffatome aufweisen.

3. Perlglanzkonzentrate nach mindestens einem der Ansprüche 1 bis 2, **dadurch gekennzeichnet, daß** sie als Komponente (b) Emulgatoren enthalten, die ausgewählt sind aus der Gruppe, die gebildet wird von :
(b1) Anlagerungsprodukten von 2 bis 30 Mol Ethylenoxid und/ oder 0 bis 5 Mol Propylenoxid an lineare Fettalkohole mit 8 bis 22 Atomen, an Fettsäuren mit 12 bis 22 Atomen, an Alkylphenole mit 8 bis 15 C-Atomen in der Alkylgruppe und an Triglyceride;
(b2) C_{12/18}-Fettsäuremono- und -diestern von Anlagerungsprodukten von 1 bis 30 Mol Ethylenoxid an Glycerin;
(b3) Glycerinmono- und -diestem und Sorbitanmono- und -diestem von gesättigten und ungesättig-ten Fettsäuren mit 6 bis 22 Kohlenstoffatomen und deren Ethylenoxidanlagerungsprodukte;
(b4) Alkylmono- und -oligoglycosiden mit 8 bis 22 Kohlenstoffatomen im Alkylrest und deren ethoxylierten Analoga;
(b5) Anlagerungsprodukten von 15 bis 60 Mol Ethylenoxid an Ricinusöl und/oder gehärtetes Ricinusöl;
(b6) Polyolestem;
(b7) Anlagerungsprodukten von 2 bis 15 Mol Ethylenoxid an Ricinusöl und/oder gehärtetes Ricinusöl;
(b8) Partialestern auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkoholen sowie Polyglucosiden;
(b9) Trialkylphosphaten;
(b10) Wollwachsalkoholen;
(b11) Polysiloxan-Polyalkyl-Polyether-Copolymeren bzw. entsprechenden Derivaten;
(b12) Mischestem aus Pentaerythrit, Fettsäuren, Citronensäure und Fettalkoholen;
(b13) Polyalkylenglycolen.

4. Perlglanzkonzentrate nach mindestens einem der Ansprüche 1 bis 3, **dadurch gekennzeichnet, daß** sie als Komponente (b) Emulgatoren vom Typ der Alkylethersulfate enthalten.

5. Perlglanzkonzentrate nach mindestens einem der Ansprüche 1 bis 4, **dadurch gekennzeichnet, daß** sie als Komponente (c) 0,1 bis 40 Gew.-% Glycerin, 1,2-Propylenglycol, Butylenglycol, Hexylenglycol und/ oder Polyethylenglycole mit einem durchschnittlichen Molekulargewicht im Bereich von 100 bis 1.000 Dalton enthalten.

6. Verfahren zur Herstellung zur Herstellung von Perlglanzkonzentraten nach Anspruch 1, **dadurch gekennzeichnet, daß** man eine Mischung aus den Komponenten (a), (b) und gegebenenfalls (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt.

7. Verfahren zur Herstellung getrübter und perlglänzender flüssiger, wäßriger Zubereitungen wasserlöslicher grenzflächenaktiver Stoffe, bei dem man den klaren wäßrigen Zubereitungen bei 0 bis 40°C Perlglanzkonzentrate nach den Ansprüchen 1 bis 5 in einer Menge von 0,5 bis 40 Gew.-% der Zubereitung zusetzt und unter Rühren darin verteilt.

8. Verwendung von Fettketonen, mit der Maßgabe, daß sie in Summe mindestens 18 Kohlenstoffatome enthalten, als Perlglanzwachse zur Herstellung von oberflächenaktiven Zubereitungen.

## Claims

1. Aqueous pearlizing concentrates containing - based on the nonaqueous component -
(a) 1 to 99.9% by weight of fatty ketones, with the proviso that they contain in all at least 18 carbon atoms,
(b) 0.1 to 90% by weight of anionic, nonionic, cationic, ampholytic and/or zwitterionic emulsifiers and
(c) 0 to 40% by weight of polyols,
with the proviso that the quantities add up to 100% by weight.

2. Pearlizing concentrates as claimed in claim 1, **characterized in that** they contain fatty ketones corresponding to formula (II):
**R**^{**2**}**-CO-R**^{**3**} **(II)**
in which R² and R³ independently of one another are optionally hydroxy-substituted alkyl and/or alkenyl groups containing 1 to 22 carbon atoms, with the proviso that they contain in all at least 18 carbon atoms,
as component (a).

3. Pearlizing concentrates as claimed in at least one of claims 1 to 2, **characterized in that** they contain as component (b) emulsifiers selected from the group consisting of:
(b1) products of the addition of 2 to 30 mol ethylene oxide and/or 0 to 5 mol propylene oxide onto linear fatty alcohols containing 8 to 22 carbon atoms, onto fatty acids containing 12 to 22 carbon atoms, onto alkylphenols containing 8 to 15 carbon atoms in the alkyl group and onto triglycerides;
(b2) C_{12/18} fatty acid monoesters and diesters of addition products of 1 to 30 mol ethylene oxide onto glycerol;
(b3) glycerol monoesters and diesters and sorbitan monoesters and diesters of saturated and unsaturated fatty acids containing 6 to 22 carbon atoms and ethylene oxide adducts thereof;
(b4) alkyl mono- and oligoglycosides containing 8 to 22 carbon atoms in the alkyl group and ethoxylated analogs thereof;
(b5) products of the addition of 15 to 60 mol ethylene oxide onto castor oil and/or hydrogenated castor oil;
(b6) polyol esters;
(b7) products of the addition of 2 to 15 mol ethylene oxide onto castor oil and/or hydrogenated castor oil;
(b8) partial esters based on linear, branched, unsaturated or saturated C_{12/22} fatty acids, ricinoleic acid and also 12-hydroxystearic acid and glycerol, polyglycerol, pentaerythritol, dipentaerythritol, sugar alcohols and polyglucosides;
(b9) trialkyl phosphates;
(b10) wool wax alcohols;
(b11) polysiloxane/polyalkyl polyether copolymers and corresponding derivatives thereof;
(b12) mixed esters of pentaerythritol, fatty acids, citric acid and fatty alcohols;
(b13) polyalkylene glycols.

4. Pearlizing concentrates as claimed in at least one of claims 1 to 3, **characterized in that** they contain emulsifiers of the alkyl ether sulfate type as component (b).

5. Pearlizing concentrates as claimed in at least one of claims 1 to 4, **characterized in that** they contain 0.1 to 40% by weight of glycerol, 1,2-propylene glycol, butylene glycol, hexylene glycol and/or polyethylene glycols with an average molecular weight of 100 to 1,000 dalton as component (c).

6. A process for the production of the pearlizing concentrates claimed in claim 1, **characterized in that** a mixture of components (a), (b) and optionally (c) is prepared, heated to a temperature 1 to 30°C above the melting point of the mixture, mixed with the necessary quantity of water having substantially the same temperature and then cooled to room temperature.

7. A process for the production of opaque and pearlescent liquid water-containing preparations of water-soluble surface-active substances, in which the pearlizing concentrates claimed in claims 1 to 5 are added to the clear aqueous preparations at 0 to 40°C in a quantity of 0.5 to 40% by weight, based on the preparation, and distributed therein by stirring.

8. The use of fatty ketones, with the proviso that they contain in all at least 18 carbon atoms, as pearlizing waxes for the production of surface-active formulations.

## Revendications

1. Concentrés aqueux à éclat nacré renfermant - rapporté à la fraction non aqueuse -
a) de 1 à 99,9 % en poids de cétones grasses étant précisé qu'elles contiennent globalement au moins 18 atomes de carbone,
b) de 0,1 à 90 % en poids d'agents émulsionnants anioniques, non ioniques, cationiques, ampholytiques et/ou zwitterioniques ainsi que
c) de 0 à 40 % en poids de polyols,
étant précise que les données en quantités se complètent à 100 % en poids.

2. Concentrés à éclat nacré aqueux selon la revendication 1,
**caractérisés en ce qu'**
ils renferment comme composant (a) des cétones grasses de formule (II)
R² - CO - R³ (II)
dans laquelle R² et R³ représentent indépendamment l'un de l'autre, des restes alkyle et/ou alcényle ayant de 1 à 22 atomes de carbone éventuellement hydroxy substitués, étant précisé qu'ils possèdent globalement au moins 18 atomes de carbone.

3. Concentrés à éclat nacré selon au moins une des revendications 1 à 2,
**caractérisés en ce qu'**
ils renferment comme composant (b) des agents émulsionnants choisis dans le groupe formé
(b1) des produits d'addition de 2 à 30 moles d'oxyde d'éthylène et/ou de 0 à 5 moles d'oxyde de propylène sur des alcools gras linéaires ayant de 8 à 22 atomes de carbone, sur des acides gras ayant de 12 à 22 atomes de carbone, sur des alkylphénols ayant de 8 à 15 atomes de carbone dans le groupe alkyle et sur des triglycérides,
(b2) des mono- et des diesters d'acide gras en C_{12/}C₁₈ de produits d'addition de 1 à 30 moles d'oxyde d'éthylène sur le glycérol,
(b3) des mono- et des diesters de glycérol et des mono et des diesters de sorbitol et d'acides gras saturés et insaturés ayant de 6 à 22 atomes de carbone et de leurs produits d'addition d'oxyde d'éthylène,
(b4) des alkyl mono- et oligoglycosides ayant de 8 à 22 atomes de carbone dans le reste alkyle et leurs analogues éthoxylés,
(b5) des produits d'addition de 15 à 60 moles d'oxyde d'éthylène sur l'huile de ricin et/ou de l'huile de ricin durcie,
(b6) des esters de polyol,
(b7) des produits d'addition de 2 à 15 moles d'oxyde d'éthylène sur de l'huile de ricin et/ou de l'huile de ricin durcie,
(b8) des esters partiels à base d'acides gras en C₁₂/C₂₂ linéaires, ramifiés, non saturés ou saturés, d'acide ricinoléique ainsi que d'acide 12-hydroxystéarique et de glycérol, de polyglycérol, de pentaérythritol, de dipentaérythritol, d'alcools de sucre ainsi que de polyglucosides,
(b9) des phosphates de trialkyle,
(b10)d'alcool de cire de laine,
(b11)des copolymères polysiloxane - polyalkyle - polyéther ou des dérivés correspondants,
(b12)des esters mixtes de pentaérythritol, d'acides gras, d'acide citrique et d'alcools gras,
(b13)des polyalkylène glycols.

4. Concentrés à éclat nacré selon au moins une des revendications 1 à 3,
**caractérisés en ce qu'**
ils renferment comme composant (b) des agents émulsionnants du type des alkyléthersulfates.

5. Concentrés à éclat nacré selon au moins l'une des revendications 1 à 4,
**caractérisés en ce qu'**
ils renferment comme composant (c) de 0,1 à 40 % en poids de glycérol, de 1,2-propylèneglycol, de butylèneglycol, d'hexylèneglycol, et/ou de polyéthylèneglycol ayant un poids moléculaire moyen dans la zone de 100 à 1000 Daltons.

6. Procédé de préparation de concentrés à éclat nacré selon la revendication 1,
**caractérisé en ce qu'**
on prépare un mélange des composants (a) et (b) et éventuellement (c), on le chauffe à une température qui se situe de 1 à 30°C au-dessus de son point de fusion, on le mélange avec la quantité nécessaire d'eau environ à la même température et ensuite on le refroidit à la température ambiante.

7. Procédé d'obtention de préparations aqueuses opalescentes, et à éclat nacré, liquides, de substances solubles dans l'eau, tensioactives, selon lequel on ajoute aux préparations aqueuses limpides à 0 à 40°C des concentrés à éclat nacré selon les revendications 1 à 5, en quantité allant de 0,5 à 40 % en poids de la préparation et on les y répartit par agitation.

8. Utilisation de cétones grasses, étant précisé qu'elles renferment globalement au moins 18 atomes de carbone, comme cires à éclat pour l'obtention de préparations tensioactives.
